# EUROPÄISCHE PATENTSCHRIFT

(11) **EP 0 682 008 B1**
(45) Veröffentlichungstag und Bekanntmachung des Hinweises auf die Patenterteilung: **30.12.1998**
(21) Anmeldenummer: 95106356.9
(22) Anmeldetag: 27.04.1995
(51) Int. Cl.: C07C 205/12, C07C 211/52, C07C 25/13, C07C 255/50, C07C 201/08, C07C 209/36, C07C 17/093, C07C 253/00

(54) **Substituierte 2-Chlor-3,4,5-trifluorbenzole und ein Verfahren zu ihrer Herstellung**
Substituted 2-chloro-3,4,5-trifluorobenzenes and process for their preparation
Chloro-2 trifluoro-3,4,5 benzènes et procédé pour leur préparation

(30) Priorität: 09.05.1994 DE 4416329
(43) Veröffentlichungstag der Anmeldung: 15.11.1995
(73) Patentinhaber: Clariant GmbH, 65929 Frankfurt am Main (DE)
(72) Erfinder: Pfirmann, Ralf, Dr., D-64347 Griesheim (DE); Schach, Thomas, Dr., D-64579 Gernsheim (DE)

(56) Entgegenhaltungen:
- EP-A- 0 447 259
- EP-A- 0 600 317
- DE-A- 4 123 322
- CHEMICAL ABSTRACTS, Band 117, Nr. 23, 7. Dezember 1992, Columbus, Ohio, USA OHASHI M. et al. "Preparation of 2-chloro-3,4,5-trifluoro- benzoic acids" Seite 829, Spalte 1, Nr. 233 606b; & JP-A-04 187 656

## Beschreibung

Die vorliegende Erfindung betrifft neue, substituierte 2-Chlor-3,4,5-trifluorbenzole, nämlich 2-Chlor-3,4,5-trifluornitrobenzol, 2-Chlor-3,4,5-trifluoranilin, 2-Chlor-3,4,5-trifluorbenzonitril und 2-Chlor-3,4,5-trifluorchlorbenzol sowie ein Verfahren zu ihrer Herstellung.

Die erfindungsgemäßen neuen Verbindungen stellen wertvolle Zwischenprodukte für die Herstellung von modernen und hochwirksamen antibakteriellen Mitteln aus der Reihe der 6-Fluor-8-halogenchinoloncarbonsäuren dar.

6-Fluor-8-halogenchinoloncarbonsäuren lassen sich nach literaturbekannten Methoden (EP-A-0 198 192, EP-A-0 357 047) ausgehend von 2,3-Dihalogen-4,5-difluorbenzoesäuren herstellen. Dabei ist es unwesentlich, welche Art von Halogenatom in der für den erforderlichen Ringschluß bedeutsamen 2-Position in der 2,3-Dihalogen-4,5-difluorbenzoesäure steht. Ein Fluoratom in der 2-Position besitzt den Vorteil, daß es leicht substituierbar ist, aber die Einführung eines Fluoratoms in diese Position ist stets mit einem erhöhten technischen Aufwand und dementsprechend mit Kosten verbunden. Daher könnte es jedoch von besonderem Interesse sein, anstelle eines Fluoratoms ein Chloratom in der 2-Position unter Ringschluß zu substituieren.

Bislang wird für die Herstellung von 6,8-Difluorchinolonarbonsäuren 2,3,4,5-Tetrafluorbenzoesäure als Ausgangsmaterial eingesetzt. Die Herstellung von 2,3,4,5-Tetrafluorbenzoesäure ist beispielsweise in der EP-A-0 510 490 oder EP-A-0 140 482 beschrieben. Nachteil dieser Varianten der Herstellung ist, daß in Abhängigkeit des gewählten Herstellungsweges stets aufwendige, vielstufige Synthesesequenzen zu durchlaufen sind und es wegen der unvermeidbaren Bildung von Fluorwasserstoff, der im Verlauf der Synthese freigesetzt wird, zwangsweise Korrosionsprobleme auftreten.

2-Chlor-3,4,5-trifluorbenzoesäure, die durch Umsetzung von 1-Chlor-2,3,4-trifluorbenzol mit CCl₄ und AlCl₃ und nachfolgende Hydrolyse des 2-Chlor-3,4,5-trifluorbenzotrichlorid herstellbar ist, dient als Zwischenprodukt zur Herstellung von 6,7,8-Trifluorchinolincarbonsäuren.

Es besteht somit ein Bedarf nach neuen Zwischenprodukten, die eine einfache Herstellung von 2-Chlor-3,4,5-trifluorbenzoesaure ermöglichen. Diese neuen Zwischenprodukte sollen sich ausgehend von leicht zuganglichen und in technischen Mengen verfügbaren Ausgangsstoffen in einfacher Weise und mit hohen Ausbeuten herstellen lassen. Zudem soll auch sichergestellt sein, daß die 2-Chlor-3,4,5-trifluor-benzoesaure ohne großen Aufwand mit guter Ausbeute und zugleich in hoher Reinheit erhalten wird.

Gelöst wird diese Aufgabe durch substituierte 2-Chlor-3,4,5-trifluorbenzole der allgemeinen Formel 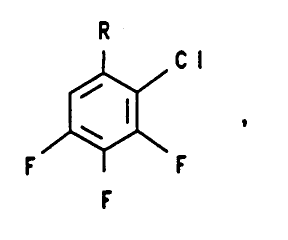 worin R für NO₂, NH₂, CN oder Cl steht.

Die vorliegende Erfindung betrifft somit die Verbindungen 2-Chlor-3,4,5-trifluornitrobenzol, 2-Chlor-3,4,5-trifluoranilin, 2-Chlor-3,4,5-trifluorbenzonitril, und 2-Chlor-3,4,5-trifluorchlorbenzol, insbesondere 2-Chlor-3,4,5-trifluornitrobenzol, 2-Chlor-3,4,5-trifluoranilin und 2-Chlor-3,4,5-trifluorbenzonitril, bevorzugt 2-Chlor-3,4,5-trifluornitrobenzol und 2-Chlor-3,4,5-trifluorbenzonitril, besonders bevorzugt 2-Chlor-3,4,5-trifluorbenzonitril.

Zu der für die Herstellung von 6,8-Difluorchinolincarbonsäuren benötigten 2-Chlor-3,4,5-trifluorbenzoesäure gelangt man, indem man 2-Chlor-3,4,5-trifluornitrobenzol in das 2-Chlor-3,4,5-trifluoranilin und dieses in das 2-Chlor-3,4,5-trifluorbenzonitril überführt und aus dem 2-Chlor-3,4,5-trifluorbenzonitril, beispielsweise durch Verseifen, die 2-Chlor-3,4,5-trifluorbenzoesäure herstellt. Es ist allerdings auch möglich, 2-Chlor-3,4,5-trifluornitrobenzol über das 2-Chlor-3,4,5-trifluoranilin in das 2-Chlor-3,4,5-trifluorchlorbenzol oder 2-Chlor-3,4,5-trifluorbrombenzol umzuwandeln und aus dem 2-Chlor-3,4,5-trifluorchlorbenzol oder dem 2-Chlor-3,4,5-trifluorbrombenzol direkt oder nach Umwandlung in das 2-Chlor-3,4,5-trifluorbenzonitril die gewünschte 2-Chlor-3,4,5-trifluorbenzoesäure zu gewinnen.

Die vorliegende Erfindung betrifft ferner ein Verfahren zur Herstellung von substituierten 2-Chlor-3,4,5-trifluorbenzolen der allgemeinen Formel 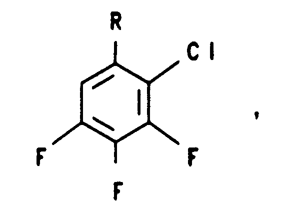 worin R für NO₂, NH₂, CN, Cl oder Br steht. Es ist dadurch gekennzeichnet, daß man 2,3,4-Trifluorchlorbenzol mit einem Nitrierungsmittel in Anwesenheit oder Abwesenheit eines wasserunlöslichen Lösungsmittels bei -10 bis 50 °C umsetzt, 2-Chlor-3,4,5-trifluornitrobenzol oder ein 2-Chlor-3,4,5-trifluornitrobenzol und 5-Chlor-2,3,4-trifluornitrobenzol enthaltendes Gemisch in Anwesenheit eines organischen Solvens reduziert, 2-Chlor-3,4,5-trifluoranilin oder ein 2-Chlor-3,4,5-trifluoranilin und 5-Chlor-2,3,4-trifluoranilin enthaltendes Gemisch isoliert, dieses gegebenenfalls diazotiert und die diazotierte Gruppe gegen ein Chloridatom, Bromidatom oder einen Cyanidrest austauscht.

Ein Vorteil des erfindungsgemäßen Verfahrens besteht darin, daß man 2,3,4-Trifluorchlorbenzol als Ausgangsmaterial verwenden kann. 2,3,4-Trifluorchlorbenzol läßt sich auf vergleichweise einfache Art aus einem bereits in technischen Mengen verfügbaren Ausgangsstoff herstellen. Man setzt nämlich das vergleichsweise leicht zugängliche 2,3,4-Trifluornitrobenzol unter den Bedingungen der denitrierenden Chlorierung zum gewünschten 2,3,4-Trifluorchlorbenzol um. Die Reaktion verläuft glatt und liefert das gewünschte 2,3,4-Trifluorchlorbenzol in guter Ausbeute.

Unter den Bedingungen einer elektrophilen Substitution (beispielsweise Friedel-Crafts Acylierung oder Bromierung) erhält man aus 2,3,4-Trifluorchlorbenzol stets ein Gemisch verschiedener Isomeren, nämlich das entsprechende (erwünschte) in 2-Stellung chlorierte Produkt und das (unerwünschte) Produkt, bei dem das Chloratom in der 5-Position steht. So liefert eine Acylierung mittels Chloracetylchlorid lediglich 31 % 2-Chlor-3,4,5-trifluorchloracetophenon aber nicht weniger als 69 % unerwünschtes 5-Chlor-2,3,4-chloracetophenon (DE-OS 41 23 322). Bromiert man 2,3,4-Trifluorchlorbenzol so erhält man ein Gemisch, das 2-Chlor-3,4,5-trifluorbrombenzol und 5-Chlor-2,3,4-trifluorbrombenzol etwa in Verhältnis 50:50 enthält (siehe auch Vergleichsbeispiel).
Vor diesem Hintergrund ist als überraschend anzusehen, daß die erfindungsgemäße Arbeitsweise mittels einer Nitrierung unter vergleichsweise recht milden Bedingungen ein Gemisch liefert, in dem das erwünschte 2-Chlor-3,4,5-trifluornitrobenzol mit 75 % und mehr das Hauptprodukt darstellt, während das unerwünschte 5-Chlor-2,3,4-trifluornitrobenzol mit lediglich 25 % und weniger als Nebenprodukt anfällt. Da das 2-Chlor-3,4,5-trifluornitrobenzol sich durch Reduktion in das entsprechende 2-Chlor-3,4,5-trifluoranilin und dieses sich diazotieren und anschließend über eine Sandmeyer-Reaktion in das entsprechende 2-Chlor-3,4,5-trifluorchlorbenzol und 2-Chlor-3,4,5-trifluorbrombenzol überführen läßt, eröffnet die vorliegende Erfindung auch einen recht selektiven Zugang sowohl zu 2-Chlor-3,4,5-trifluorchlorbenzol als auch zu 2-Chlor-3,4,5-trifluorbrombenzol.

In der nachfolgenden Abbildung sind die einzelnen Reaktionsschritte einschließlich einer möglichen Wiederverarbeitung einzelner Reaktionsprodukte schematisch wiedergegeben. 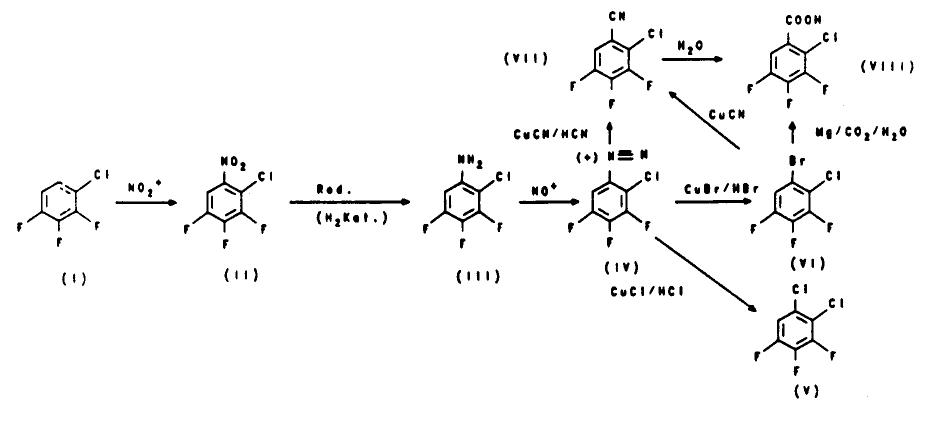
- (I): = 2,3,4-Trifluorchlorbenzol
- (II): = 2-Chlor-3,4,5-trifluornitrobenzol
- (III): = 2-Chlor-3,4,5-trifluoranilin
- (IV): = 2-Chlor-3,4,5-trifluordiazonium Kation
- (V): = 2-Chlor-3,4,5-trifluorchlorbenzol (1,2-Dichlor-3,4,5-trifluorbenzol)
- (VI): = 2-Chlor-3,4,5-trifluorbrombenzol
- (VII): = 2-Chlor-3,4,5-trifluorbenzonitril
- (VIII): = 2-Chlor-3,4,5-trifluorbenzoesäure

Man setzt zunächst 2,3,4-Trifluorchlorbenzol mit einem Nitrierungsmittel, das als eigentliches Nitrierungsagens NO₂⁺ enthält oder bildet, um. Das Nitrierungsmittel enthält üblicherweise Salpetersäure, NO, NO₂, N₂O₃ und/oder N₂O₄, gegebenenfalls gelöst in Schwefelsäure oder Oleum. Gut geeignet als Nitriermittel ist Salpetersäure, die in Schwefelsäure oder Oleum gelöst ist. Im allgemeinen lassen sich Nitriermittel, die 2 bis 100, insbesondere 10 bis 65, bevorzugt 15 bis 40 Gew.-% Salpetersäure enthalten, mit gutem Erfolg verwenden.

Man setzt zweckmäßigerweise das Nitrierungsmittel entsprechend einem Mol-Verhältnis Salpetersäure:2,3,4-Trifluorchlorbenzol von (0,5 bis 2,5):1, insbesondere (1 bis 1,5):1, bevorzugt (1,05 bis 1,2):1 ein. In diesem Zusammenhang sei darauf aufmerksam gemacht, daß NO, NO₂, N₂O₃ und/oder N₂O₄, insbesondere NO₂, N₂O₃ und/oder N₂O₄ in Anwesenheit von Wasser in einem gewissen Umfange auch Salpetersäure bilden.

Die Nitrierung des 2,3,4-Trifluorchlorbenzol läßt sich - wie zuvor bereits erwähnt - in Abwesenheit oder Anwesenheit eines wasserunlöslichen Lösungsmittels durchführen. Als wasserunlösliches Lösungsmittel gebraucht man ein unter den Reaktionsbedingungen der Nitrierung inertes organisches Lösungsmittel. Hierfür sind generell einfach oder mehrfach chlorierte aliphatische Kohlenwasserstoffe oder Gemische derselben zu empfehlen, insbesondere einfach oder mehrfache chlorierte aliphatische Kohlenwasserstoffe mit 1 bis 8 Kohlenstoffatomen sowie deren Mischungen.

Gut geeignet als wasserunlösliches Lösungsmittel sind Dichlormethan, Chloroform, Tetrachlormethan, 1,2-Dichlorethan, einfach oder mehrfach chlorierte aliphatische Kohlenwasserstoffe mit 1 bis 4 Kohlenstoffatomen oder Gemische dieser Verbindungen.

Im allgemeinen spielt die Menge an wasserunlöslichen Lösungsmittel keine große Rolle. In den meisten Fällen genügt es, das wasserunlösliche Lösungmittel und 2,3,4-Trifluorchlorbenzol im Gewichtsverhältnis (0,2 bis 10):1, insbesondere (1 bis 7,5):1, bevorzugt (1,5 bis 5):1 einzusetzen.

Wie bereits erwähnt, läßt sich die Nitrierung bei vergleichsweise niedrigen Temperaturen durchführen. Üblicherweise setzt man 2,3,4-Trifluorchlorbenzol mit dem Nitrierungsmittel bei -5 bis 40, insbesondere 0 bis 30°C um. Es lassen sich auch Temperaturen oberhalb 40°C anwenden, jedoch wird dadurch kein Vorteil erzielt. Bei Temperaturen unterhalb von -5°C kann man die Nitrierung auch ablaufen lassen, muß dafür aber vergleichsweise lange Reaktionszeit in Kauf nehmen.

Durch die Nitrierung des 2,3,4-Trifluorchlorbenzols erhält man ein 2-Chlor-3,4,5-trifluornitrobenzol und 5-Chlor-2,3,4-trifluornitrobenzol enthaltendes Gemisch. Man kann vorteilhafterweise dieses Gemisch, ohne es in einem separaten Schritt aufzutrennen, für die nachfolgende Stufe der Reduktion verwenden. Bei dieser Arbeitsweise führt man die Abtrennung des unerwünschten 5-Chlor-2,3,4-trifluorbenzolderivates zu einem späteren Zeitpunkt, beispielsweise auf der Stufe des Anilins, Nitrils, der Brom- oder Chlorverbindung, insbesondere auf der Stufe des Anilins oder Nitrils durch. Es ist allerdings auch möglich, das 2-Chlor-3,4,5-trifluornitrobenzol und 5-Chlor-2,3,4-trifluornitrobenzol enthaltende Gemisch beispielsweise durch fraktionierende Destillation, Extraktivdestillation, Schmelzkristallisation, Umkristallisation oder Chromatographie oder durch eine Kombination dieser Methoden aufzuarbeiten und in die nachfoglende Stufe der Reduktion 2-Chlor-3,4,5-trifluornitrobenzol einzusetzen.

Nach Beendigung der Nitrierung versetzt man das Nitriergemisch gegebenenfalls mit kaltem Wasser, um eventuell noch vorhandene Schwefelsäure zu verdünnen, und gibt anschließend, um das nitrierte Produktgemisch zum entsprechenden Amingemisch zu reduzieren, ein gegenüber den Bedingungen der Reduktion inertes organisches Solvens zu. Geeignet als organisches Solvens sind aliphatische Kohlenwasserstoffe, cycloaliphatische Kohlenwasserstoffe, aromatische Kohlenwasserstoffe, aliphatische Alkohole mit 1 bis 6 Kohlenstoffatomen oder Alkylester aliphatischer Carbonsäuren, gebildet aus Alkanolen mit 1 bis 6 Kohlenstoffatomen und aliphatischen Carbonsäuren mit 1 bis 6 Kohlenstoffatomen. Man führt zweckmäßigerweise die Reduktion in Anwesenheit eines dieser organischen Solventien oder in Anwesenheit einer Mischung der vorstehend genannten organischen Solventien durch.

Als Beispiele für geeignete organische Lösungsmittel seien - ohne Anspruch auf Vollständigkeit zu erheben - Hexan, Methylcyclohexan, Toluol, o-Xylol, m-Xylol, p-Xylol, Gemische isomerer Xylole, Methanol, Ethanol, n-Propanol, i-Propanol, n-Butanol, i-Butanol, Essigsäuremethylester, Essigsäureethylester, Essigsäurebutylester und/oder Butoxylessigsäure-3-methoxy-butylester, insbesondere Toluol, o-Xylol, m-Xylol, p-Xylol, Gemische isomerer Xylole, Methanol, Essigsäureethylester und/oder Essigsäurebutylester genannt. Es lassen sich auch Mischung zweier oder mehrerer dieser organischen Lösungsmittel verwenden.

Die Reduktion der Nitrogruppe in die entsprechende Aminogruppe läßt sich üblicherweise, beispielsweise in Houben-Weyl, Methoden der organischen Chemie, Band X1/1, Seiten 360 bis 490 beschriebene Verfahren durchführen. Besonders einfach gestaltet sich die Reduktion, wenn man in Anwesenheit eines Hydrierkatalysators mittels Wasserstoff oder mittels Hydrazin reduziert, oder Eisen, eine niederwertige Schwefelverbindung, beispielsweise H₂S, ein Polysulfid oder Dithionit, Zinn, Zinn(II)chlorid, Eisen(II)hydroxid oder einen Wasserstoffdonor als Reduktionsmittel verwendet.

Als Hydrierkatalysator eignen sich die üblichen, ein Übergangsmetall enthaltenden Katalysatoren, die ein Trägermaterial enthalten oder auch trägerfrei sein können.

Üblicherweise führt man die Reduktion mittels Wasserstoff bei einem Druck von 0,05 bis 20, insbesondere 0,1 bis 10, bevorzugt 0,2 bis 8 MPa in Anwesenheit eines Nickel, Platin oder Palladium und gegebenenfalls einen Träger enthaltenden Hydrierkatalysators bei 0 bis 100, insbesondere 40 bis 90 °C durch. Als Trägermaterial dienen beispielsweise Aluminiumoxid, Bimsstein, Aktivkohle, Kieselsäure, Kieselgur, Kieselgel und SiO₂. Es lassen sich auch Gemische der vorstehend genannten Trägerstoffe verwenden.

Als Hydrierkatalysatoren können beispielsweise Raney-Nickel, Ni auf Kieselgur oder Kieselsäure, Pd und/oder Pt auf Aluminiumoxid, Bimsstein oder Aktivkohle, insbesondere Pd auf Aktivkohle und Pt auf Aktivkohle, bevorzugt Pt auf Aktivkohle in Betracht. Man verwendet die Nickelkatalysatoren in Mengen von 0,5 bis 5, insbesondere 1 bis 4 Massen% (gerechnet als Nickel) und die Edelmetallkatalysatoren in Mengen zwischen 0,05 bis 3, insbesondere 0,3 bis 1 Gew.-% (gerechnet als reines Übergangsmetall), bezogen jeweils auf zu hydrierendes Produkt. Die Katalysatoren können gegebenenfalls durch geeignete Zusatzstoffe, beispielsweise Amine oder BaSO₄, oder durch geeignete Behandlungsmethoden, beispielsweise partielle Vergiftung, modifiziert werden.

Bei Einsatz von Eisen als Reduktionsmittel arbeitet man in der Regel unter Zusatz einer Säure, insbesondere einer Mineralsäure, wie HCl, H₂SO₄ oder H₃PO₄. Alternativ ist auch die Methode der sogenannten Transfer-Hydrierung (Houben-Weyl, Methoden der organischen Chemi Band IV/1c Seiten 67 bis 76) anwendbar. Hierbei werden anstelle von Wasserstoff Wasserstoffdonoren eingesetzt.

Brauchbar als Wasserstoffdonor sind beipsielsweise Cyclohexadien-(1,3), Cyclohexen oder Hydrazin.

Nach Beendigung der Reduktion (Hydrierung) wird der Katalysator, beispielsweise durch Filtration des heißen Reaktionsproduktes, abgetrennt, das Reaktionsprodukt eingeengt und durch anschließende Filtration oder Fraktionierung gewonnen. Dabei empfiehlt es sich, wegen der Empfindlichkeit der erhaltenen Anilinderivate gegen Luft unter Zusatz von Antioxidantien, beispielsweise Hydrazin, Hydraziniumsalzen oder 2,6-Di-tert.-butyl-4-methylphenol, zu arbeiten.

Üblicherweise isoliert man nach Beendigung der Reduktion das 2-Chlor-3,4,5-trifluoranilin und 5-Chlor-2,3,4-trifluoranilin enthaltende Gemisch oder das 2-Chlor-3,4,5-trifluoranilin durch Destillation, Schmelzkristallisation, Chromatographie oder durch einfache Phasentrennung (Extraktion). Die Art der Isolierung richtet sich an der Art des Einsatzproduktes und der Art des gewünschten Endproduktes sowie am geforderten Reinheitsgrad des Endproduktes aus. Beabsichtigt man, ein 2-Chlor-3,4,5-trifluoranilin und 5-Chlor-2,3,4-trifluoranilin enthaltendes Gemisch zu isolieren, so empfiehlt es sich, das reduzierte Rohgemisch mittels einer einfachen Destillation aufzuarbeiten.

Will man hingegen aus einem 2-Chlor-3,4,5-trifluoranilin und 5-Chlor-2,3,4-trifluoranilin enthaltenden Gemisch reines 2-Chlor-3,4,5-trifluoranilin isolieren, so empfiehlt es sich, das reduzierte Rohgemisch mittels einer fraktionierenden Destillation aufzuarbeiten.

Anschließend wird das 2-Chlor-3,4,5-trifluoranilin und 5-Chlor-2,3,4-trifluoranilin enthaltende Gemisch oder das 2-Chlor-3,4,5-trifluoranilin mittels salpetriger Säure oder einer salpetrige Säure bildenden Substanz bei -20 bis 50, insbesondere -10 bis 30°C diazotiert. Als eigentliches Diazotierungsagens ist NO⁺, das sich aus salpetriger Säure bildet, anzusehen.
Man setzt 5-Chlor-2,3,4-trifluoranilin und/oder 2-Chlor-3,4,5-trifluoranilin und die salpetrige Säure im Molverhältnis 1:(0,9 bis 10), insbesondere 1:(1 bis 5), bevorzugt 1:(1,05 bis 2) ein.
Als Diazotierungsmittel dienen beispielsweise Nitrosylschwefelsäure und Alkalinitrite oder Erdalkalinitrite in Mineralsäuren, wie Salzsäure, Schwefelsäure, Bromwasserstoffsäure und Jodwasserstoffsäure, aus denen salpetrige Säure gebildet wird.

Infolge der Diazotierung entsteht aus der ursprünglichen Aminogruppe eine diazotierte Gruppe (Diazoniumgruppe). In einem nachfolgenden Schritt wird die diazotierte Gruppe (Diazoniumgruppe) mit einem Alkalichlorid, Alkalibromid, Alkalicyanid, Erdalkalichlorid, Erdalkalibromid und/oder Erdalkalicyanid in Anwesenheit von Kupfer oder einem Kupfersalz unter Austausch gegen ein Chloratom, Bromatom oder einen Cyanidrest umgesetzt (Sandmeyer-Reaktion).

Man erhält auf diese Weise das gewünschte 2-Chlor-3,4,5-trifluorchlorbenzol (1,2-Dichlor-3,4,5-trifluorbenzol), 2-Chlor-3,4,5-trifluorbrombenzol und 2-Chlor-3,4,5-trifluorbenzonitril, das entweder direkt durch Austausch der diazotierten Gruppe gegen einen Cyanidrest oder durch Brom-Cyan-Austausch aus dem 2-Chlor-3,4,5-trifluorbrombenzol hergestellt werden kann. Das 2-Chlor-3,4,5-trifluorbenzonitril kann auf einfache Art und Weise mittels einer Hydrolyse in die 2-Chlor-3,4,5-benzoesäure umgewandelt werden, die ihrerseits - wie bereits eingangs erläutert - zur Herstellung von 6,8-Difluorchinoloncarbonsäuren benutzt werden kann.

Die einzelnen Reaktionsschritte lassen sich unter Atmosphärendruck, unter Unterdruck oder Überdruck durchführen.

Die substituierten 2-Chlor-3,4,5-trifluorbenzole der allgemeinen Formel 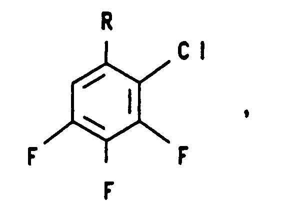 worin R für NO₂, NH₂, CN oder Cl steht, lassen sich als Ausgangsstoffe zur Herstellung von 2-Chlor-3,4,5-trifluorbenzoesäure verwenden.

Die nachfolgenden Beispiele belegen die Erfindung.

### Beispiel 1

### 2-Chlor-3,4,5-trifluornitrobenzol

54,9 g (0.33 Mol) 2,3,4-Trifluorchlorbenzol werden in 47,2 g (0,11 Mol) 20 %igem Oleum vorgelegt und bei 21 °C in 1 Stunde unter Kühlung 64,8 g (0,35 Mol) Mischsäure, hergestellt aus 68,8 Gew.-% 100 %iger H₂SO₄ + 31,2 Gew.-% 100 %iger HNO₃, zugetropft. Man rührt 30 min nach und hydrolysiert mit 330 g Eiswasser. Die Phasen werden getrennt (57,6 g organische Phase) und die wäßrige Phase wird mit 65 g Toluol ausgeschüttet, es verbleiben 429,1 g gelbe wäßrige Phase und 66,2 g gelbe Toluol-Phase. Die vereinigten organischen Phasen werden zweimal mit 50 g 10 %iger Natriumcarbonat-Lösung und anschließend dreimal mit 75 g Wasser gewaschen. Man erhält 117,7 g organische Phase, darin sind 58,8 g (0.278 Mol, 84 %) Chlortrifluornitrobenzole enthalten, die durch fraktionierte Destillation getrennt (Isomerenverhältnis 2-Chlor-3,4,5-trifluornitrobenzol: 5-Chlor-2,3,4-trifluorbenzol (NMR) = 76:24), aber auch in Mischung weiterverarbeitet werden können, wobei die Isomerentrennung auf einer späteren Stufe erfolgt.

Arbeitet man mit 0,15 Mol 2,3,4-Trifluorchlorbenzol in 50 g 100 %iger Schwefelsäure als Verdünnungsmittel und nitriert mit 30,5 g (0,165 Mol) Mischsäure hergestellt aus 68,8 Gew.-% 100 %iger H₂SO₄ und 31,2 Gew.-% 100 %iger HNO₃ bei 5 bis 12°C, so ist nach 1 Stunde Dosierdauer und 1 Stunde Nachrührzeit die Umsetzung beendet. Man erhält im wesentlichen dasselbe Ergebnis.

### 2-Chlor-3,4,5-trifluornitrobenzol:

¹H-NMR (CDCl₃, TMS):
- δ =: 7.76 (q, 1H, J = 2.25 Hz, J = 6.84 Hz, J = 9.16 Hz, Ar-H⁶)

¹⁹F-NMR (CDCl₃, CFCl₃):
- δ =: -127.08 (q, 1F, J=2.25 Hz, J=7.4 Hz, J=19.7 Hz, Ar-F³)
-131.17 (q, 1F, J=7.4 Hz, J=9.16 Hz, J=20.8 Hz, Ar-F⁵)
-147.34 (q, 1F, J=6.84 Hz, J=19.7 Hz, J=20.8 Hz, Ar-F⁴)
- MS: m/z (%) =: 61 (12.3), 68 (6.7), 9 (8.6), 75 (7.3), 79 (10.1), 80 (28.7), 91 (7.9), 93 (6.8), 99 (23.6), 111 (7.8), 115 (81.9), 117 (28), 130 (29.7), 153 (41.9), 155 (14.3), 165 (63.3), 167 (21.3), 181 (37.9), 183 (12.3), 211 (M⁺, 100), 212 (7.4), 213 (33)

### 5-Chlor-2,3,4-trifluornitrobenzol:

¹H-NMR (CDCl₃, TMS):
- δ =: 8.07 (q, 1H, J=2.44 Hz, J=6.83 Hz, J=7.03 Hz, Ar-H⁶)

¹⁹F-NMR (CDCl₃, CFCl₃)
- δ =: -121.52 (q, 1F, J = 6.83 Hz, J = 14.6 Hz, J = 19.32 Hz, Ar-F⁴)
-127.12 (q, 1F, J = 7.03 Hz, J = 14.6 Hz, J = 20.6 Hz, Ar-F²)
-150.64 (q, 1F, J = 2.44 Hz, J = 19.32 Hz, J = 20.6 Hz, Ar-F³)
- MS: m/z (%) =: 61 (14.7), 80 (37.2), 99 (30), 115 (97.2), 117 (34.2), 130 (36.2), 153 (65.6), 155 (21.7), 165 (61.9), 167 (20.4), 181 (44.2), 183 (16.3), 211 (M⁺,100), 213 (32.7)

### Beispiel 2

### 2-Chlor-3,4,5-trifluoranilin

117,7 g des Rohproduktes aus Beispiel 1 (toluolische Lösung von 2-Chlor-3,4,5-trifluornitrobenzol und 5-Chlor-2,3,4-trifluornitrobenzol) werden mit 0,7 g sulfitiertem Pt/C-Kontakt bei 40°C vorgelegt. Unter leichtem Wasserstoffüberdruck wird die Reaktionsmischung auf 80°C erwärmt. Nach 5 Stunden ist die Reduktion beendet, durch GC lassen sich Nitroverbindungen nicht mehr nachweisen. Der Katalysator wird abfiltriert, mit 75 g Toluol gewaschen und die wäßrige Phase (5,6 g) vom Filtrat abgetrennt. Der Hauptteil des Toluols wird bei Normaldruck abdestilliert und der verbleibende Rückstand bei 1 mbar/80°C destilliert. Man erhält 44,0 g (0,242 Mol. 87 %) eines Gemisches der beiden isomeren Chlor-trifluoraniline (2-Chlor-3,4,5-trifluoranilin und 5-Chlor-2,3,4-trifluoranilin) als farblosen Festkörper (die Aniline sind im Destillat im Verhältnis 78:22 enthalten). Auch die Aniline können durch fraktionierte Destillation getrennt werden, wobei das gewünschte 2-Chlor-3,4,5-trifluoranilin zuerst übergeht.

### 2-Chlor-3,4,5-trifluoranilin:

IR: ν [cm⁻¹] = 680, 825, 880, 1045, 1075, 1125, 1200, 1250, 1470, 1520, 1615, 1645, 3430, 3520.

¹H-NMR (CDCl₃, TMS):
- δ =: 6.38 (q, 1H, J = 2.3 Hz, J = 6.7 Hz, J = 11.45 Hz, Ar-H⁶)

¹⁹F-NMR (CDCl₃, CFCl₃):
- δ =: -134.51 (q, 1F, J = 2.3 Hz, J = 6.1 Hz, J = 21.2 Hz, Ar-F³)
-136.73 (q, 1F, J = 6.1 Hz, J = 11.45 Hz, J = 21.8 Hz, Ar-F⁵)
-172.09 (q, 1F, J = 6.7 Hz, J = 21.2 Hz, J = 21.8 Hz, Ar-F⁴)
- MS: m/z (%) =: 68 (3.8), 69 (6.9), 70 (4.4), 75 (7.3), 76 (3.8), 79 (2.7), 80 (3.41), 91 (3.9), 93 (3.7), 94 (3.7), 96 (2.1), 99 (13.7), 100 (2.8), 118 (9.7), 119 (33.8), 125 (2.8), 126 (16.0), 144 (2.61), 145 (10.5), 146 (11.9), 153 (8.7), 154, (3.3), 155 (3.1), 161 (2.8), 181 (M⁺, 100), 182 (7.9), 183 (32.8)

### 5-Chlor-2,3,4-trifluoranilin:

IR: ν [cm⁻¹]= 705, 870, 1020, 1170, 1225, 1260, 1515, 1615, 1645, 3420, 3510

¹H-NMR (CDCl₃, TMS):
- δ =: 6.56 (q, 1H, J = 2.45 Hz, J = 6.5 Hz, J = 8.25 Hz, Ar-H⁶)

¹⁹F-NMR (CDCl₃, CFCl₃):
- δ =: -149.52 (q, 1F, J = 1.0 Hz, J = 6.5 Hz, J = 21.0 Hz, Ar-F^{4.2})
-157.13 (q, 1F, J = 1.0 Hz, J = 8.25 Hz, J = 19.45 Hz, Ar-F^{2.4})
-157.81 (q, 1F, J = 2.45 Hz, J = 19.45 Hz, J = 21.0, Ar-F³)

### Beispiel 3 (Vergleichsbeispiel)

99,9 g (0,6 Mol) 2,3,4-Trifluorchlorbenzol werden mit 4 g (72 mMol) Eisen bei 100°C vorgelegt und es werden binnen 3,5 Stunden 111,9 g (0.7 Mol) Brom zugetropft. Die Mischung wird danach bis zu einer Gesamtreaktionszeit von 24 Stunden bei Rückfluß nachgerührt. Nach dem Abkühlen wird der Kolbeninhalt vorsichtig solange mit wäßriger 10 %iger Natriumsulfit-Lösung versetzt, bis die die Lösung keine Oxidationswirkung mehr zeigt. Die organische Phase wird abgetrennt, über Magnesiumsulfat getrocknet, filtriert und fraktioniert destilliert (Einsatz 127,2 g). Im Rohprodukt sind 2-Chlor-3,4,5-trifluorbrombenzol und 5-Chlor-2,3,4-trifluorbrombenzol im Verhältnis 59:41 vorhanden. In der organischen Phase sind neben 18 % Ausgangsmaterial und 68 % Monobromverbindungen auch 13,5 % Dibromverbindungen (alle GC, a/a) vorhanden. Man erhält 74,7 g (0,337 Mol, 56,6 %) Gemisch der Monobromverbindungen mit einem Reingehalt > 96 % und dem angegebenen Isomerenverhältnis. Unter Einbeziehung der in den Zwischen- und Vorläufen enthaltenden Mengen werden 86,1 g (65 %) Monobrom-chlor-trifluorbenzole im Gemisch erhalten.

### Beispiel 4

### 2-Chlor-3,4,5-trifluorbenzonitril

94,5 g (0,59 Mol) Kupfersulfat werden in 206 g Wasser gelöst. Diese Lösung wird unter Rühren zu einer aus 155 g Wasser, 65,0 g (1,0 Mol) Kaliumcyanid und 30,0 g (0,44 Mol) 25 %iger Ammoniak-Lösung bereiteten Lösung gegeben. Dabei wird durch ein Eisbad gekühlt. Man erhält eine dunkelbraune bis dunkelgrüne Lösung.

In einem anderen Gefäß werden 92,6 g (0,51 Mol) 2-Chlor-3,4,5-trifluoranilin in 200 g 30 %ige Salzsäure gegeben, wobei unter Zugabe von 100 g Eis gekühlt wird. Die Temperatur wird durch Außenkühlung auf -10°C eingestellt. Dazu tropft man unter Rühren und Kühlen 36,2 g (0,52 Mol) Natriumnitrit in 60 g Wasser. Die zähflüssig werdende, gelbe Reaktionsmischung wird mit Wasser auf 2 l aufgefüllt und nach Ende der Dosierung 15 min nachgerührt.

Die zunächst hergestellte kupferhaltige Lösung wird auf Eis gegeben und anschließend setzt man zu dieser Mischung die Diazoniumsalz-Lösung zu. Nach vollständiger Dosierung wird die entstandene Mischung mittels 25 %iger wäßriger Ammoniak-Lösung auf pH 8 gestellt.

Man erhält durch Extraktion mit Dichlormethan eine organische Phase, die neben 2-Chlor-3,4,5-trifluorbenzonitril (62,3 %) noch 10,4 % des eingesetzten Anilins enthält. Das Anilin wird durch mehrfaches Waschen mit 10 %iger Salzsäure entfernt und die verbleibende Lösung wird fraktioniert destilliert. Man erhält 54,3 g (0,283 Mol, 56 %) 2-Chlor-3,4,5-trifluorbenzonitril als leicht gelblichen Festkörper (Reingehalt (GC) > 95 %).

### 2-Chlor-2,3,4-trifluorbenzoesäurenitril:

¹H-NMR (CDCl₃, TMS):
- δ =: 7,77 (ddd, 1H, J_{AD} = 2,3 Hz, J_{CD} = 7,6 Hz, J_{BD} = 10,3 Hz, Ar-H6)

¹⁹F-NMR (CDCl₃, CFCl₃):
- δ =: -129,2 (ddd, 1F, J_{AD} = 2,3 Hz, J_{AB} = 7,3 Hz, J_{AC} = 20,5 Hz, Ar-F3)
-134,1 (ddd, 1F, J_{AB} = 7,3 Hz, J_{BD} = 10,3 Hz, J_{BC} = 20,7 Hz, Ar-F5)
-148,8 (ddd, 1F, J_{CD} = 7,36Hz, J_{AC} = 20,5 Hz, J_{BC} = 20,7 Hz, Ar-F4)

Die Umsetzung verläuft analog, sofern man anstatt des weitgehend isomerenreinen Anilins die Isomerenmischung einsetzt (siehe auch in Beispiel 5).

### Beispiel 5

### 1,2-Dichlor-3,4,5-trifluorbenzol

Aus einer Lösung von 82 g (0,2 Mol) Kupfersulfat-5-hydrat wird durch Zugabe von 20 g (0,16 Mol) Natriumsulfit und 40 g Natriumchlorid Kupfer(I)chlorid gefällt und in 100 ml konz. Salzsäure gelöst. Zu dieser Lösung werden unter Rühren 48,3 g (0,266 Mol) 2-Chlor-3,4,5-trifluoranilin/5-Chlor-2,3,4-trifluoranilin (Verhältnis nach NMR 78:22) gegeben. Die Mischung wird auf 10°C gekühlt und eine Lösung von 30 g (0,352 Mol) Kaliumnitrit in 60 g Wasser tropfenweise zugegeben. Während der Zugabe wird die Temperatur der Mischung unterhalb 20°C gehalten. Nach Ende der Dosierung wird 1 Stunde nachgerührt und danach 30 min auf 80°C erhitzt, nachfolgend abgekühlt und mit Natronlauge alkalisch gestellt. Durch Wasserdampfdestillation wird das Produktionsgemisch vorgereinigt. Die sich im Destillat abscheidende ölige Phase wird abgetrennt und fraktioniert destilliert (41 g). Man erhält 32,3 g (0,161 Mol, 60 %) eines 75:25 Gemisches aus 1,2-Dichlor-3,4,5-trifluorbenzol und 1,5-Dichlor-2,3,4-trifluorbenzol.

## Patentansprüche

1. Substituierte 2-Chlor-3,4,5-trifluorbenzole der allgemeinen Formel 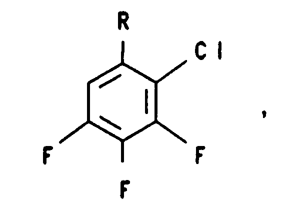 worin R für NO₂, NH₂, CN oder Cl steht.

2. Verfahren zur Herstellung von substituierten 2-Chlor-3,4,5-trifluorbenzolen der allgemeinen Formel 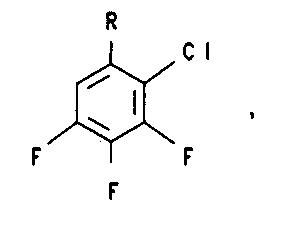 worin R für NO₂, NH₂, CN, Cl oder Br steht, dadurch gekennzeichnet, daß man 2,3,4-Trifluorchlorbenzol mit einem Nitrierungsmittel in Anwesenheit oder Abwesenheit eines wasserunlöslichen Lösungsmittels bei -10 bis 50 °C umsetzt, 2-Chlor-3,4,5-trifluornitrobenzol oder ein 2-Chlor-3,4,5-trifluornitrobenzol und 5-Chlor-2,3,4-trifluornitrobenzol enthaltendes Gemisch in Anwesenheit eines organischen Solvens reduziert, 2-Chlor-3,4,5-trifluoranilin oder ein 2-Chlor-3,4,5-trifluoranilin und 5-Chlor-2,3,4-trifluoranilin enthaltendes Gemisch isoliert, dieses gegebenfalls diazotiert und die diazotierte Gruppe gegen ein Chloridatom, Bromidatom oder einen Cyanidrest austauscht.

3. Verfahren nach Anspruch 2, dadurch gekennzeichnet, daß das Nitrierungsmittel Salpetersäure, NO, NO₂, N₂O₃ und/oder N₂O₄ gegebenenfalls gelöst in Schwefelsäure oder Oleum, enthält.

4. Verfahren nach Anspruch 2 oder 3, dadurch gekennzeichnet, daß das Nitrierungsmittel 2 bis 100, insbesondere 10 bis 65, bevorzugt 15 bis 40 Gew.-% Salpetersäure enthält.

5. Verfahren nach einem oder mehreren der Ansprüche 2 bis 4, dadurch gekennzeichnet, daß man das Nitrierungsmittel entsprechend einem Molverhältnis Salpetersäure: 2,3,4-Trifluorchlorbenzol von (0,5 bis 2,5):1, insbesondere (1 bis 1,5):1, bevorzugt (1,05 bis 1,2):1 einsetzt.

6. Verfahren nach einem oder mehreren der Ansprüche 2 bis 5, dadurch gekennzeichnet, daß man als wasserunlösliches Lösungsmittel ein gegenüber dem Nitrierungsmittel unter den Reaktionsbedingungen inertes organisches Lösungsmittel einsetzt.

7. Verfahren nach einem oder mehreren der Ansprüche 2 bis 6, dadurch gekennzeichnet, daß man als wasserunlösliches Lösungsmittel einen einfach oder mehrfach chlorierten aliphatischen Kohlenwasserstoff oder ein Gemisch derselben einsetzt.

8. Verfahren nach einem oder mehreren der Ansprüche 2 bis 7, dadurch gekennzeichnet, daß man als wasserunlösliches Lösungsmittel Dichlormethan, Chloroform, Tetrachlormethan, 1,2-Dichlorethan, einfach oder mehrfach chlorierte aliphatische Kohlenwasserstoffe mit 1 bis 4 Kohlenstoffatomen oder Gemische dieser Verbindungen einsetzt.

9. Verfahren nach einem oder mehreren der Ansprüche 2 bis 8, dadurch gekennzeichnet, daß man wasserunlösliches Lösungsmittel und 2,3,4-Trifluorchlorbenzol im Gewichtsverhältnis von (0,2 bis 10):1, insbesondere (1 bis 7,5):1, bevorzugt (1,5 bis 5):1 einsetzt.

10. Verfahren nach einem oder mehreren der Ansprüche 2 bis 9, dadurch gekennzeichnet, daß man 2,3,4-Trifluorchlorbenzol mit dem Nitrierungsmittel bei -5 bis 40, insbesondere 0 bis 30 °C umsetzt.

11. Verfahren nach einem oder mehreren der Ansprüche 2 bis 10, dadurch gekennzeichnet, daß man in Anwesenheit eines aliphatischen, cycloaliphatischen oder aromatischen Kohlenwasserstoffs, eines aliphatischen Alkohols mit 1 bis 6 Kohlenstoffatomen oder eines Alkylesters aliphatischer Carbonsäuren, gebildet aus Alkanolen mit 1 bis 6 Kohlenstoffatomen und aliphatischen Carbonsäuren mit 1 bis 6 Kohlenstoffatomen, als organisches Solvens reduziert.

12. Verfahren nach einem oder mehreren der Ansprüche 2 bis 11, dadurch gekennzeichnet, daß man Hexan, Methycyclohexan, Toluol, Xylol, Methanol, Ethanol, n-Propanol, i-Propanol, n-Butanol, i-Butanol, Essigsäuremethylester, Essigsäureethylester, Essigsäurebutylester und/oder Butoxyl-essigsäure-3-methoxy-butylester als organisches Solvens einsetzt.

13. Verfahren nach einem oder mehreren der Ansprüche 2 bis 12, dadurch gekennzeichnet, daß man mittels Wasserstoff oder Hydrazin in Gegenwart eines Hydrierkatalysators, mittels Eisen, Hydrazin oder mittels eines Wasserstoffdonors reduziert.

14. Verfahren nach einem oder mehreren der Ansprüche 2 bis 13, dadurch gekennzeichnet, daß man mittels Wasserstoff bei einem Druck von 0,05 bis 20 insbesondere 0,1 bis 10 MPa in Anwesenheit eines Nickel, Platin oder Palladium und gegebenenfalls einen Träger enthaltenden Hydrierkatalysators bei 0 bis 100, insbesondere 40 bis 90°C reduziert.

15. Verfahren nach einem oder mehreren der Ansprüche 2 bis 13, dadurch gekennzeichnet, daß man mittels Cyclohexadien-(1,3), Cyclohexen oder Hydrazin als Wasserstoffdonor reduziert.

16. Verfahren nach einem oder mehreren der Ansprüche 2 bis 13, dadurch gekennzeichnet, daß man mittels Hydrazin oder mittels eines Wasserstoffdonors bei 0 bis 100°, insbesondere 20 bis 80°C, bevorzugt 40 bis 75°C reduziert.

17. Verfahren nach einem oder mehreren der Ansprüche 2 bis 16, dadurch gekennzeichnet, daß man das 2-Chlor-3,4,5-trifluoranilin und 5-Chlor-2,3,4-trifluoranilin enthaltende Gemisch oder das 2-Chlor-3,4,5-trifluoranilin durch Destillation, Schmelzkristallisation oder Chromatographie oder durch einfache Phasentrennung isoliert.

18. Verfahren nach einem oder mehreren der Ansprüche 2 bis 17, dadurch gekennzeichnet, daß man das 2-Chlor-3,4,5-trifluoranilin oder das 2-Chlor-3,4,5-trifluoranilin und 5-Chlor-2,3,4-trifluoranilin enthaltende Gemisch mittels salpetriger Säure oder einer salpetriger Säure bildenden Substanz bei -20 bis 50°, insbesondere -10 bis 80°C diazotiert.

19. Verfahren nach einem oder mehreren der Ansprüche 2 bis 18, dadurch gekennzeichnet, daß man 5-Chlor-2,3,4-trifluoranilin und/oder 2-Chlor-3,4,5-trifluoranilin und die salpetrige Säure im Molverhältnis 1:(0,9 bis 10), insbesondere 1:(1 bis 5), bevorzugt 1:(1,05 bis 2) einsetzt.

20. Verfahren nach einen oder mehreren der Ansprüche 2 bis 19, dadurch gekennzeichnet, daß man die diazotierte Gruppe mit einem Alkali- und/oder Erdalkalichlorid, -bromid oder -cyanid in Anwesenheit von Kupfe oder einem Kupfersalz unter Austausch gegen ein Chloridatom, Bromidatom oder einen Cyanidrest umsetzt.

21. Verwendung der substituierten 2-Chlor-3,4,5-trifluorbenzole der allgemeinen Formel 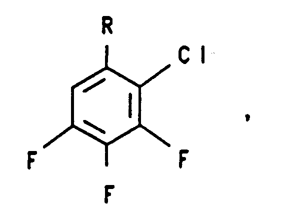 worin R für NO₂, NH₂, CN oder Cl steht als Ausgangsstoffe zur Herstellung von 2-Chlor-3,4,5-trifluorbenzoesäure.

## Claims

1. A substituted 2-chloro-3,4,5-trifluorobenzene of the formula 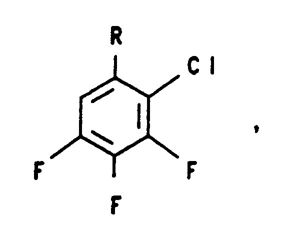 in which R is NO₂, NH₂, CN or Cl.

2. A process for the preparation of a substituted 2-chloro-3,4,5-trifluorobenzene of the formula 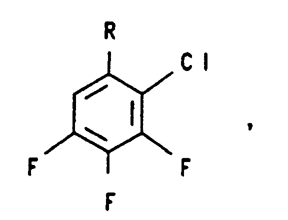 in which R is NO₂, NH₂, CN, Cl or Br, which comprises reacting 2,3,4-trifluorochlorobenzene with a nitrating agent in the presence or absence of a water-insoluble solvent at from -10 to 50°C, reducing 2-chloro-3,4,5-trifluoronitrobenzene or a mixture comprising 2-chloro-3,4,5-trifluoronitrobenzene and 5-chloro-2,3,4-trifluoronitrobenzene in the presence of an organic solvent, isolating 2-chloro-3,4,5-trifluoroaniline or a mixture comprising 2-chloro-3,4,5-trifluoroaniline and 5-chloro-2,3,4-trifluoroaniline, diazotizing this product, if desired, and exchanging the diazotized group for a chloride atom, bromide atom or a cyanide radical.

3. The process as claimed in claim 2, wherein the nitrating agent contains nitric acid, NO, NO₂, N₂O₃ and/or N₂O₄, dissolved if desired in sulfuric acid or oleum.

4. The process as claimed in claim 2 or 3, wherein the nitrating agent contains from 2 to 100 % by weight, in particular from 10 to 65 % by weight and preferably from 15 to 40 % by weight of nitric acid.

5. The process as claimed in one or more of claims 2 to 4, wherein the nitrating agent is employed in accordance with a molar ratio of nitric acid to 2,3,4-trifluorochlorobenzene of (from 0.5 to 2.5):1, in particular (from 1 to 1.5):1 and preferably (from 1.05 to 1.2):1.

6. The process as claimed in one or more of claims 2 to 5, wherein an organic solvent which is inert to the nitrating agent under the reaction conditions is employed as water-insoluble solvent.

7. The process as claimed in one or more of claims 2 to 6, wherein a mono- or polychlorinated aliphatic hydrocarbon or a mixture thereof is employed as water-insoluble solvent.

8. The process as claimed in one or more of claims 2 to 7, wherein dichloromethane, chloroform, tetrachloromethane, 1,2-dichloroethane, a mono- or polychlorinated aliphatic hydrocarbon of 1 to 4 carbon atoms, or a mixture of these compounds, is employed as water-insoluble solvent.

9. The process as claimed in one or more of claims 2 to 8, wherein water-insoluble solvent and 2,3,4-trifluorochlorobenzene are employed in a weight ratio of (from 0.2 to 10):1, in particular (from 1 to 7.5):1 and preferably (from 1.5 to 5):1.

10. The process as claimed in one or more of claims 2 to 9, wherein 2,3,4-trifluorochlorobenzene is reacted with the nitrating agent at from -5 to 40°C, in particular from 0 to 30°C.

11. The process as claimed in one or more of claims 2 to 10, wherein reduction is carried out in the presence of an aliphatic, cycloaliphatic or aromatic hydrocarbon, an aliphatic alcohol of 1 to 6 carbon atoms or an alkyl ester of an aliphatic carboxylic acid, formed from alkanols of 1 to 6 carbon atoms and aliphatic carboxylic acids of 1 to 6 carbon atoms, as organic solvent.

12. The process as claimed in one or more of claims 2 to 11, wherein hexane, methylcyclohexane, toluene, xylene, methanol, ethanol, n-propanol, isopropanol, n-butanol, isobutanol, methyl acetate, ethyl acetate, butyl acetate and/or 3-methoxybutyl butoxyacetate are/is employed as organic solvent.

13. The process as claimed in one or more of claims 2 to 12, wherein reduction is carried out using hydrogen or hydrazine in the presence of a hydrogenation catalyst, using iron, hydrazine or using a hydrogen donor.

14. The process as claimed in one or more of claims 2 to 13, wherein reduction is carried out using hydrogen at a pressure of from 0.05 to 20 MPa, in particular from 0.1 to 10 MPa, in the presence of a hydrogenation catalyst which comprises nickel, platinum or palladium and, if desired, a support, at from 0 to 100°C, in particular from 40 to 90°C.

15. The process as claimed in one or more of claims 2 to 13, wherein reduction is carried out using 1,3-cyclohexadiene, cyclohexene or hydrazine as hydrogen donor.

16. The process as claimed in one or more of claims 2 to 13, wherein reduction is carried out using hydrazine or using a hydrogen donor at from 0 to 100°C, in particular from 20 to 80°C and preferably from 40 to 75°C.

17. The process as claimed in one or more of claims 2 to 16, wherein the mixture comprising 2-chloro-3,4,5-trifluoroaniline and 5-chloro-2,3,4-trifluoroaniline or the 2-chloro-3,4,5-trifluoroaniline is isolated by distillation, melt crystallization or chromatography or by simple phase separation.

18. The process as claimed in one or more of claims 2 to 17, wherein the 2-chloro-3,4,5-trifluoroaniline or the mixture comprising 2-chloro-3,4,5-trifluoroaniline and 5-chloro-2,3,4-trifluoroaniline is diazotized using nitrous acid, or a substance which forms nitrous acid, at from -20 to 50°C, in particular from -10 to 80°C.

19. The process as claimed in one or more of claims 2 to 18, wherein 5-chloro-2,3,4-trifluoroaniline and/or 2-chloro-3,4,5-trifluoroaniline and the nitrous acid are employed in a molar ratio of 1:(from 0.9 to 10), in particular 1:(from 1 to 5) and preferably 1:(from 1.05 to 2).

20. The process as claimed in one or more of claims 2 to 19, wherein the diazotized group is reacted with an alkali metal chloride, bromide or cyanide and/or an alkaline earth metal chloride, bromide or cyanide in the presence of copper or a copper salt and is exchanged for a chloride atom, bromide atom or a cyanide radical.

21. The use of a substituted 2-chloro-3,4,5-trifluorobenzene of the formula 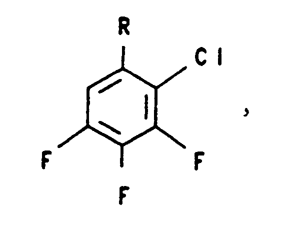 in which R is NO₂, NH₂, CN or Cl, as a starting material for the preparation of 2-chloro-3,4,5-trifluorobenzoic acid.

## Revendications

1. 2-chloro-3,4,5-trifluorobenzènes substitués de formule générale 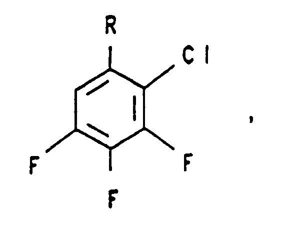 dans laquelle R représente NO₂, NH₂, CN ou Cl.

2. Procédé de préparation de 2-chloro-3,4,5-trifluorobenzènes substitués de formule générale 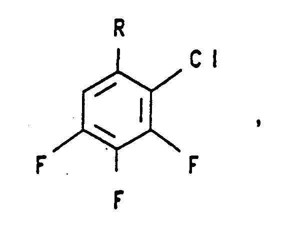 dans laquelle R représente NO₂, NH₂, CN, Cl ou Br, caractérisé en ce que l'on met à réagir le 2,3,4-trifluorochlorobenzène avec un agent de nitration en présence ou en l'absence d'un solvant insoluble dans l'eau entre -10°C et 50°C, l'on réduit le 2-chloro-3,4,5-trifluoronitrobenzène ou un mélange contenant du 2-chloro-3,4,5-trifluoronitrobenzène et du 5-chloro-2,3,4-trifluoronitrobenzène en présence d'un solvant organique, l'on isole la 2-chloro-3,4,5-trifluoroaniline ou un mélange contenant la 2-chloro-3,4,5-trifluoroaniline et la 5-chloro-2,3,4-trifluoroaniline, l'on soumet celui-ci éventuellement à une diazotation et on échange le groupe diazoté avec un atome donnant un chlorure, un atome donnant un bromure ou un groupe cyanure.

3. Procédé selon la revendication 2, caractérisé en ce que l'agent de nitration contient de l'acide nitrique, NO, NO₂, N₂O₃ et/ou N₂O₄, éventuellement dissous dans de l'acide sulfurique ou de l'oléum.

4. Procédé selon la revendication 2 ou 3, caractérisé en ce que l'agent de nitration contient 2 à 100, en particulier 10 à 65, de préférence 15 à 40% en poids d'acide nitrique.

5. Procédé selon une ou plusieurs des revendications 2 à 4, caractérisé en ce que l'on utilise l'agent de nitration correspondant à un rapport molaire acide nitrique : 2,3,4-trifluorochlorobenzène de (0,5 à 2,5):1, en particulier de (1 à 1,5):1, de préférence de (1,05 à 1,2):1.

6. Procédé selon une ou plusieurs des revendications 2 à 5, caractérisé en ce que l'on utilise comme solvant insoluble dans l'eau un solvant organique inerte vis à vis de l'agent de nitration dans les conditions de réaction.

7. Procédé selon une ou plusieurs des revendications 2 à 6, caractérisé en ce que l'on utilise comme solvant insoluble dans l'eau un hydrocarbure aliphatique une ou plusieurs fois chloré ou un mélange de ceux-ci.

8. Procédé selon une ou plusieurs des revendications 2 à 7, caractérisé en ce que l'on utilise comme solvant insoluble dans l'eau le dichlorométhane, le chloroforme, le tétrachlorométhane, le 1,2-dichloroéthane, les hydrocarbures aliphatiques une ou plusieurs fois chlorés ayant 1 à 4 atomes de carbone ou des mélanges de ces composés.

9. Procédé selon une ou plusieurs des revendications 2 à 8, caractérisé en ce que l'on utilise un solvant insoluble dans l'eau et le 2,3,4-trifluorochlorobenzène dans le rapport en poids de (0,2 à 10):1, en particulier de (1 à 7,5):1, de préférence de (1,5 à 5):1.

10. Procédé selon une ou plusieurs des revendications 2 à 9, caractérisé en ce que l'on met à réagir le 2,3,4-trifluorochlorobenzène avec l'agent de nitration de -5 jusqu'à 40, en particulier de 0 à 30°C.

11. Procédé selon une ou plusieurs des revendications 2 à 10, caractérisé en ce que l'on réalise une réduction en présence, comme solvant organique, d'un hydrocarbure aliphatique, cycloaliphatique ou aromatique, d'un alcool aliphatique avec 1 à 6 atomes de carbone ou d'un ester alkylique d'acides carboxyliques, formé à partir d'alcanols ayant 1 à 6 atomes de carbone avec des acides carboxyliques aliphatiques avec 1 à 6 atomes de carbone.

12. Procédé selon une ou plusieurs des revendications 2 à 11, caractérisé en ce que l'on utilise comme solvant organique l'hexane, le méthylcyclohexane, le toluène, le xylène, le méthanol, l'éthanol, le n-propanol, l'i-propanol, le n-butanol, l'i-butanol, l'acétate de méthyle, l'acétate d'éthyle, l'acétate de butyle et/ou le butoxylacétate de 3-méthoxybutyle.

13. Procédé selon une ou plusieurs des revendications 2 à 12, caractérisé en ce que l'on réalise la réduction au moyen d'hydrogène ou d'hydrazine en présence d'un catalyseur d'hydrogénation, au moyen de fer, d'hydrazine ou au moyen d'un donneur d'hydrogène.

14. Procédé selon une ou plusieurs des revendications 2 à 13, caractérisé en ce que l'on réalise la réduction avec de l'hydrogène à une pression de 0,05 à 20, en particulier de 0,1 à 10 MPa en présence d'un catalyseur d'hydrogénation contenant du nickel, du platine ou du palladium et éventuellement un support, de 0 à 100, en particulier de 40 à 90°C.

15. Procédé selon une ou plusieurs des revendications 2 à 13, caractérisé en ce que l'on réduit au moyen de cyclohexa-1,3-diène, de cyclohexène ou d'hydrazine comme donneur d'hydrogène.

16. Procédé selon une ou plusieurs des revendications 2 à 13, caractérisé en ce que l'on réduit au moyen d'hydrazine ou au moyen d'un donneur d'hydrogène de 0 à 100, en particulier de 20 à 80°C, de préférence de 40 à 75°C.

17. Procédé selon une ou plusieurs des revendications 2 à 16, caractérisé en ce que l'on isole le mélange contenant la 2-chloro-3,4,5-trifluoroaniline et la 5-chloro-2,3,4-trifluoroaniline ou la 2-chloro-3,4,5-trifluoroaniline par distillation, cristallisation à l'état fondu, chromatographie ou par simple séparation de phases.

18. Procédé selon une ou plusieurs des revendications 2 à 17, caractérisé en ce que l'on effectue une diazotation de la 2-chloro-3,4,5-trifluoroaniline ou du mélange contenant la 2-chloro-3,4,5-trifluoroaniline et la 5-chloro-2,3,4-trifluoroaniline au moyen d'acide nitrique ou d'une substance formant de l'acide nitrique de -20 jusqu'à 50, en particulier de -10 jusqu'à 80°C.

19. Procédé selon une ou plusieurs des revendications 2 à 18, caractérisé en ce que l'on utilise la 5-chloro-2,3,4-trifluoroaniline et/ou la 2-chloro-3,4,5-trifluoroaniline et l'acide nitrique dans la proportion molaire de 1:(0,9 à 10), en particulier de 1:(1 à 5), de préférence de 1:(1,05 à 2).

20. Procédé selon une ou plusieurs des revendications 2 à 19, caractérisé en ce que l'on réalise l'échange du groupe diazoté contre un atome donnant un chlorure, un atome donnant un bromure ou un groupe cyanure avec un chlorure, bromure ou cyanure de métal alcalin et/ou alcalino-terreux en présence de cuivre ou d'un sel de cuivre.

21. Utilisation des 2-chloro-3,4,5-trifluoronitrobenzènes subtitués de formule générale 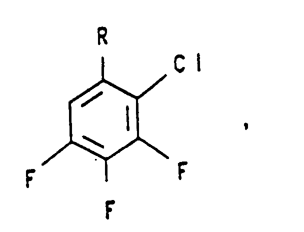 dans laquelle R représente NO₂, NH₂, CN ou Cl comme produits de départ pour la préparation d'acide 2-chloro-3,4,5-trifluorobenzoïque.
